# EUROPEAN PATENT SPECIFICATION

(11) **EP 1 646 321 B1**
(45) Date of publication and mention of the grant of the patent: **01.07.2009**
(21) Application number: 04753733.7
(22) Date of filing: 27.05.2004
(51) Int. Cl.: A61B 17/12

(54) **SYSTEM FOR ELECTRICALLY DETERMINING POSITION AND DETACHMENT OF AN IMPLANTABLE DEVICE**
SYSTEM ZUM ELEKTRISCHEN FESTSTELLEN VON POSITION UND ZUM ABLÖSEN EINER IMPLANTIERBAREN VORRICHTUNG
SYSTEME PERMETTANT DE DETERMINER PAR VOIE ELECTRIQUE LA POSITION ET LA ZONE DE DETACHEMENT D'UN DISPOSITIF IMPLANTABLE

(30) Priority: 23.07.2003 US 625196
(43) Date of publication of application: 19.04.2006
(73) Proprietor: Boston Scientific Limited, Barbados, West Indies (BB)
(72) Inventor: GUGLIELMI, Guido, I-00195 Roma (IT); EDER, Joseph, C., Los Altos Hills, CA 94024 (US)
(74) Representative: Altenburg, Udo
(86) International application number: PCT/US2004/016968
(87) International publication number: WO 2005/009252

(56) References cited:
- EP-A- 0 739 607
- EP-A- 0 830 873
- US-A- 5 669 905
- US-A1- 2003 014 073
- US-B1- 6 397 850

## Description

### FIELD OF THE INVENTION

The field of the invention pertains to implantable devices, and more particularly, to electric monitoring of the positioning and detachment of an implantable device from a delivery system.

### BACKGROUND OF THE INVENTION

In many clinical situations, blood vessels are occluded or blocked off to control bleeding, prevent blood supply to tumors, and block blood flow within an aneurysm or other vascular abnormality. Intracranial aneurysms may be particularly difficult to access and treat when they are formed in remote cerebral blood vessels. If left untreated, normal forces from blood flow through a vessel can rupture fragile tissue in the area of the aneurysm causing a stroke.

Various implants, such as vaso-ocblusive devices, have been used to treat aneurysms by decreasing blood flow to the aneurysm. A vaso-occlusive device is a surgical implant that is delivered through a catheter, which is inserted through a blood vessel and placed within or near an aneurysm. Vaso-occlusive devices tend to induce blood clotting or formation of a thrombus, which reduces blood flow to the aneurysm and limits its growth.

Radiopaque markers and fluoroscopy are typically used to track the position of the detachment zone and coil attached thereto as they are advanced through the catheter. More specifically, a radiopaque marker is placed at a distal end or tip of the catheter, and another radiopaque marker is placed towards a proximal end of the catheter. The distal marker on the catheter facilitates location of the catheter tip at the aneurysm site. The delivery wire also includes a radiopaque marker. The wire and proximal catheter markers are arranged so that the wire marker is generally aligned with the proximal catheter marker when the detachment zone of the coil extends just beyond the catheter tip. When the radiopaque markers are aligned, the coil is detached from the delivery wire at the detachment zone electrolytically or by breaking a mechanical connection.

The positioning of detachment zones and implants, however, can be improved. For example, some conventional systems do not properly position an implant, even when fluoroscopy is utilized, and minor positioning errors can impact the effectiveness of an implant. Thus, the detachment zones and devices should be monitored and positioned more accurately. Further, when multiple coil implants are delivered to an aneurysm, one coil can radiographicaly hide or obstruct other coils, thus making it more difficult to properly position a coil, resulting in positioning errors. Radiopaque markers used with angiographic visualization can also impair the positioning and effectiveness of various components. For example, proximal markers on the catheter typically make the catheter less flexible. Consequently, catheters with radiopaque markers may be less maneuverable through a vascular cavity, particularly through smaller, cranial and curved vessels. Further, catheters are often shaped with steam, which can change the distance between radiopaque catheter markers, thus impairing the ability to properly position a coil. Proximal markers on the delivery wire can also make the delivery wire less flexible, thus increasing the likelihood that the catheter tip can be moved or forced out of the aneurysm.

F-P-A-0 830 873 forms the basis for the pre-characterising section of claim 1.

### SUMMARY OF THE INVENTION

According to the present invention there is provided the system of claim 1.

In one embodiment of the invention, a system is provided for positioning an implant in a body. The implant can be a coil, such as a Guglielmi Detachable Coil (GDC). The coil can also be coated with a bio-reactive material to initiate formation of tissue in the aneurysm, or be a coil composed of a bio-reactive material or various non bioactive polymers. The implant can include platinum or another radiopaque material. The implant can also be a stent or a filter. The system includes a catheter, a delivery member, such as a delivery wire, a temporary connection joining a distal end of the delivery member to the implant, and an electrical measurement device or sensor The catheter is inserted into a vascular cavity in the body. The delivery member, the temporary connection and the implant are advanced through the catheter. The electrical measurement device detects an electrical condition related to a position of the temporary connection and the device in the catheter. The electrical condition changes when the temporary connection reaches a predetermined location, such as the distal tip of the catheter.

The electrical measurement device can detect and measure various electrical conditions or parameters, such as current, voltage, and impedance. For example, when monitoring current, the measurement device compares a reference current, such as a trickle current, with a second current that is generated when the temporary connection and implant reach or exit the distal tip of the catheter. The system can also include a visual or audio indicator that generates a signal in response to the changed electrical condition. Other control signals can also be generated to indicate a change in electrical condition.

The system may also include a power supply that is coupled to the delivery member. The power supply provides an electrical current through the delivery member and the temporary connection to electrolytically break the temporary connection by, for example, corroding or disintegrating a portion of the temporary connection. The electrical measurement device can be included within the power supply or be a separate external component. In one embodiment, an electrical circuit is completed through the delivery member, the temporary connection, the electrical measurement device, the power supply, and the body. If the temporary connection is not conductive, then a conductive wire can be connected between the electrical measurement device and the distal end of the catheter so that the electrical measurement device can detect the electrical condition through the conductive wire.

In alternative embodiments of the invention, instead of a power supply that provides current to electrolytically break the temporary connection, other detachment inducing mechanisms can be utilized, such as sources of heat and Radio Frequency (RF) to break heat or RF sensitive bonds, for example, by melting a plastic connection. In yet a further alternative embodiment, the temporary connection can be a temporary hydraulic connection that is broken when a hydraulic element is actuated.

### BRIEF DESCRIPTION OF THE DRAWINGS

Referring now to the drawings in which like reference numbers represent corresponding parts throughout:
**FIG. 1** illustrates a system according to the invention that utilizes an electrical measurement, detection device or sensor to monitor or identify the position of an implant and determine when the implant can be detached;
**FIG. 2A** is an electrical schematic that illustrates components of a system that simulates the operation of the invention, and **FIG. 2B** shows a saline-filled conductive bowl and electrical connections of **FIG. 2A** in further detail;
**FIGS 3A-E** are enlarged, microscopic images of a coil implant being advanced and detached within the saline-filled bowl of **FIGS 2A****-B;**
**FIG. 4** is an electrical schematic of components of one embodiment of the system according to the invention that includes a comparison circuit and an indication device;
**FIG. 5** is an enlarged side view of one exemplary temporary electrolytic connection that can be utilized with a system of the invention;
**FIG. 6** is an enlarged side view of one exemplary temporary mechanical connection that can be utilized with a system of the invention;
**FIG. 7A-C** are enlarged side views of a coil implant occupying different positions inside and outside of a catheter, and the manner of monitoring the positions of a temporary connection and an implant with the invention; and
**FIG. 8** is a flow diagram illustrating the monitoring a position of a temporary connection and an implant attached thereto.

### DETAILED DESCRIPTION OF THE ILLUSTRATED EMBODIMENTS

In the following description, reference is made to the accompanying drawings which form a part hereof, and which show by way of illustration specific embodiments in which the invention may be practiced. It is to be understood that other embodiments may be utilized as structural changes may be made without departing from the scope of the invention.

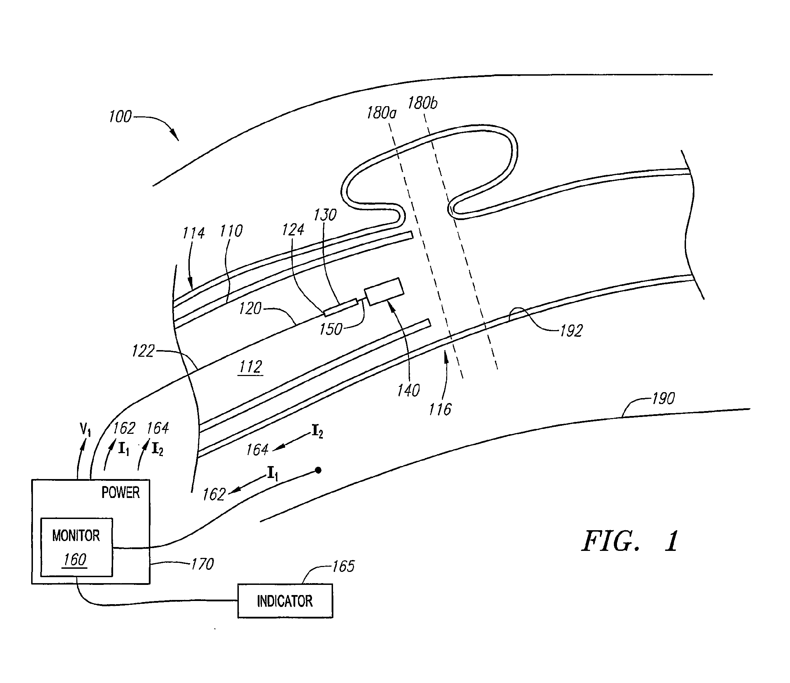

Referring to **FIG. 1****,** a system 100 according to the invention includes a catheter or sheath 110, a pusher or delivery member 120, such as a pusher wire, a fine bore tube or other tubular member (generally delivery member 120), a temporary connection or detachment zone 130, such as a temporary electrolytic, mechanical, heat-sensitive, RF-sensitive or hydraulic connection (generally temporary connection 130), an implant 140, an insulative member 150 between the conductive temporary connection 130 and the implant 140, an electrical measurement, monitoring or detection device or sensor 160, and a device that initiates breaking of the temporary connection 130, such as a power supply 170 for providing current to break an electrolytic connection. The system 100 tracks or monitors the position of the temporary connection 130 and the implant 140 attached thereto as they are advanced through the catheter 110. The system 100 determines when the temporary connection 130 reaches or passes a predetermined position 180, such as a position 180a or a position 180b (generally 180) at which the temporary connection 130 reaches or exits the distal tip of the catheter 110. Indeed, the positions 180a and 180b are merely illustrative of various positions that can be selected.

Persons of ordinary skill in the art will recognize that the invention can be utilized with various implants. For example, one exemplary implant is a vaso-occlusive device, such as a Guglielmi Detachable Coil (GDC). The coil can also be coated with a bio-reactive material to initiate formation of tissue in the aneurysm, or be a coil composed of a bio-reactive material or various non bioactive polymers. The implant can also include platinum or another radiopaque material. A further exemplary implant is a stent, such as a self expanding stent, a balloon expandable stent, a coated or a non-coated stent, a covered or partially covered stent, a high density braid stent, and a stent covered in-situ. Further, the implant can be a filter, such as a filter to capture embolic debris. In this specification, an implant refers to these exemplary implants and other suitable detachable implants that can be utilized with the invention.

The catheter 110 is made of a generally insulative or non-conductive material and defines an inner lumen or cavity 112 and has a proximal end 114 and a distal end 116. The distal end 116 is advanced through a vascular cavity or space 192, such as an artery, vessel, vein, aneurysm, arteriovenuous fistulas, or other vascular malformation in the body 190. The conductive delivery member 120 has a proximal end 122 and a distal end 124. The conductive temporary connection 130 detachably or releasably connects the distal end 124 of the delivery member 120 and the implant 140, with an insulative member 150 there between. The catheter 110 and the insulative member 150 form an "insulative chamber" that prevents or minimizes the amount of current that flows through the delivery member 120 when the member 120 is confined to the catheter lumen 112.

An initial electrical condition or parameter 162 in the circuit completed through the body 190 is detected by the measurement device or sensor 160. Exemplary electrical conditions 162 include a current, a voltage, and an impedance. While various electrical conditions 162 can be monitored, measured or detected, this specification refers to current for purposes of explanation. Further, while the measurement device 160 is shown as part of the power supply 170 in **FIG. 1**, the measurement device 160 can be separate from the power supply 170.

The magnitude of the current 162 is related to the position of the temporary connection 130 and the implant 140 attached thereto as they are pushed through the lumen 112 of the catheter 110. For example, the current 162 may indicate when the temporary connection 130 reaches or exits the distal tip 116 of the catheter 110. More specifically, the power supply 170 provides a voltage V₁ that results in a small initial or trickle current I₁ 162 flowing through the circuit completed through the patient body 190. The initial trickle current I₁ 162 results from the high resistance of the insulative catheter 110 and insulative member 150, which limit current flow when the conductive detachment zone 130 is located within the catheter 110.

As the delivery member 120, temporary connection 130 and implant 140 are pushed through the catheter lumen 112, the temporary connection 130 reaches or passes a predetermined location 180, such as the distal tip 116 of the catheter 110. As a result, the conductive temporary connection 130 exits the "insulative chamber" in the catheter 110 and contacts blood in the vascular space 192 in the body 190. Since the blood and the body 190 are conductive than the insulative elements, a larger, second current I₂ flows through the circuit formed by the delivery wire 120, the temporary connection 130, the body 190, and the measurement device 160. The measurement device 160 detects this larger, second current I₂ 164 and issues an output signal, such as an audio, visual or control signal or triggers a device to generate an audio, visual or control signal, indicating that the detachment zone 130 has reached or passed the distal tip 116 of the catheter 110. For example, a Light Emitting Diode (LED), buzzer, or a speaker can be activated in response to the changed electrical condition. In other words, the change from the smaller current I₁ 162 to the larger current I₂ 164 indicates that temporary connection 130 and implant 140 are properly positioned so that the implant 140 can be released into the aneurysm.

The output signal can be provided to a user or to a controller. For example, the output signal can indicate to a user that the temporary connection 130 and the implant 140 are properly positioned and may prompt or notify a user to manually initiate breaking of the temporary connection to detach the properly positioned detachment zone 130 and implant 140. In an alternative embodiment, an output signal can also trigger a controller to automatically initiate breaking of the temporary connection.

The implant 140 can be detached from the detachment zone or temporary connection 130 in different ways depending on the particular implant 140 and connection 130 utilized. For example, as shown in **FIG. 1****,** the power supply 170 can provide a current (e.g., a direct current (DC)) through the delivery member 120 to the temporary connection 130 to electrolytically break the connection 130, thereby releasing the implant 140 to occlude the aneurysm. The invention, however, is not limited to electrolytic temporary connections.

In an alterative embodiment, the temporary connection 130 can be mechanically broken. In yet a further alternative embodiment, the temporary connection 130 can be a heat-sensitive or Radio Frequency (RF) sensitive connection, such as a plastic coupling, that can be melted or broken when exposed to sufficient heat or RF radiation. In yet a further alternative embodiment of the invention, the temporary connection 130 is a hydraulic connection that can be broken by a hydraulic actuation device. Thus, with these alternative embodiments, instead of using current from a power supply 170 to electrolytically break a connection 130, other detachment inducing mechanisms can be utilized, such as sources of heat, RF, and hydraulic fluid. This specification, however, refers to electrolytic temporary connections and a power supply for purposes of explanation and illustration, but the invention is not so limited.

With the system 100, the position of the temporary connection 130 and the implant 140 attached thereto can be accurately monitored. Thus, the system 100 of the invention provides an accurate and predictable manner of positioning and detaching an implant 140 without resorting to radiopaque marker components and fluoroscopy tracking.

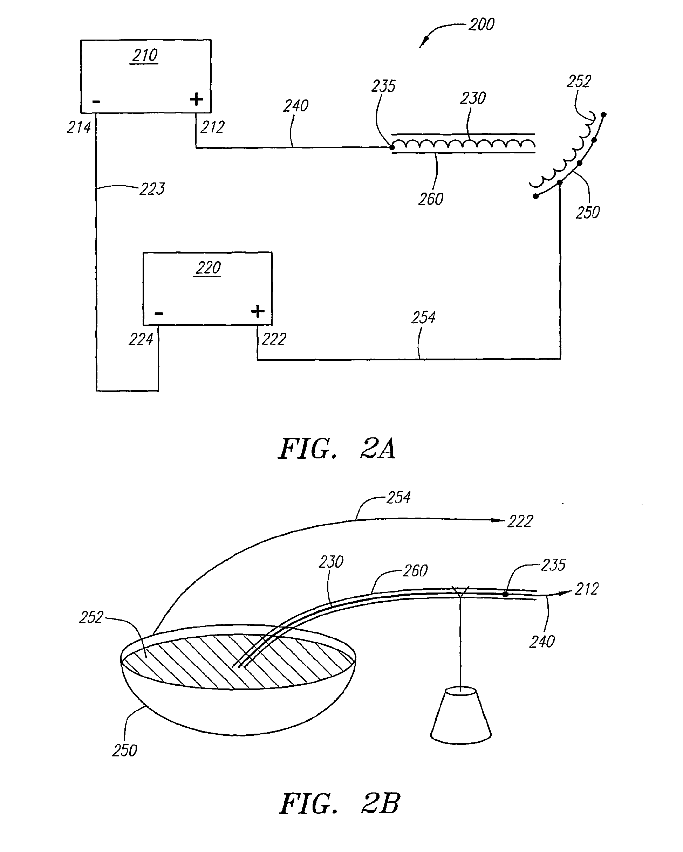

**FIGS. 2A-B** illustrate one test arrangement 200 that simulates how the invention operates when utilized in a vascular space in a body. In this test, the power supply is an alternating current (AC) generator 210, the monitoring device or sensor is a digital volt/current meter or multimeter 220 set to detect and measure AC, and the implant is a Guglielmi Detachable Coil (GDC) 230.

A conductive wire 240, which simulates a conductive temporary connection, is connected to an insulative element 235. A conductive, stainless steel bowl 250 filled with about 200 ml of saline solution 252 (or other conductive solution) simulates a vascular space with a non-insulative or conductive fluid, such as an aneurysm filled with blood.

A positive input 222 of the multimeter 220 is coupled to the bowl 250 via wire 254, a negative or ground pole 214 of the AC generator 210 is coupled a negative or ground pole 224 of the multimeter 220 via wire 223, a positive output 212 of the AC generator 210 is coupled to the proximal end of the wire 240, and the distal end of the wire 240 is connected to the insulative member 235, which is connected to the GDC 230.

The distal end of the GDC 230 was advanced through a catheter 260 into the saline 252, and the multimeter 220 detected a small trickle current of about 0.011 mA flowing through the circuit. The wire 240, insulative element 235, and GDC 230 were then advanced further into the saline 252. The GDC 230 and insulative element 235 were advanced into the saline 252 so that the conductive wire 240 exited the distal end of the catheter 260 and eventually contacted the saline 252. When saline contact occurred, the current increased from the initial trickle current of about 0.011 mA to a second, larger current of about 1.122 mA. The second, larger current resulted from reduced resistance as a result of the conductive wire 240 contacting the saline 252. In other words, the insulative element 235 no longer inhibited the current, thus permitting a larger current to flow through the circuit. This test was conducted with various AC voltage and frequency settings to verify these results, for example, the AC generator 210 was set to 300 mV at a frequency of 90 kHz.

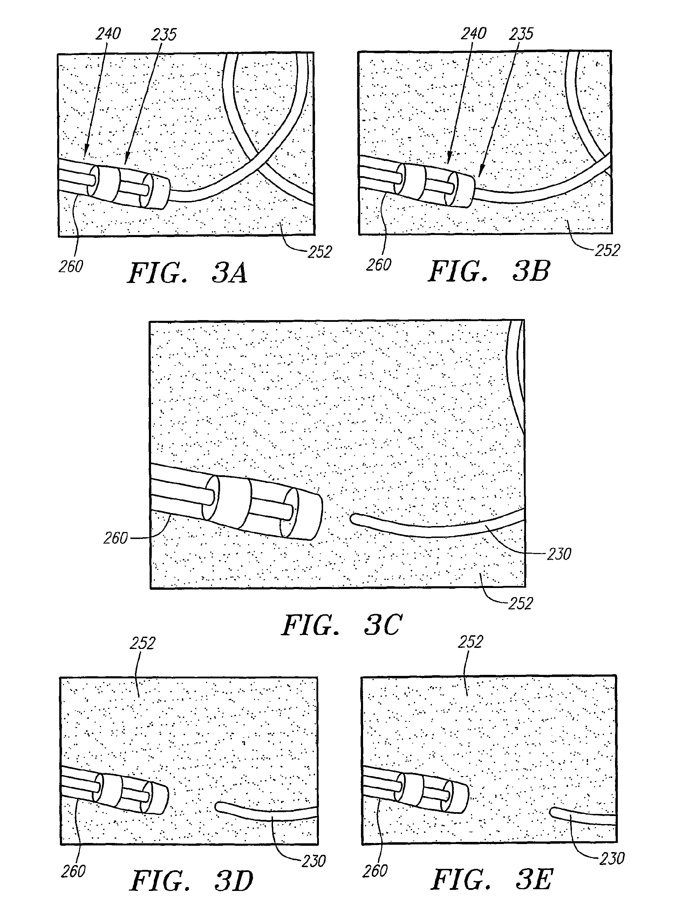

**FIGS. 3A-E** show the wire 240, insulating element 235, and GDC 230 components being advanced through the catheter 260 and into the saline 252. The distal end of the catheter 260 was submerged in the saline 252 so that as the components contacted the saline 252 as they exited the distal tip of the catheter 260. The advancement of the components was observed under a microscope.

**FIGS. 3A-B** show the GDC 230 being advanced into the saline 252, but not so far that the conductive wire 240 contacted the saline 252. As a result, only the low, trickle current of about 0.011 mA flows through the circuit due to the resistance of the insulative member 235 and catheter 210. As shown in **FIGS. 3C-E**, as the components were advanced further, the conductive wire 240 eventually exited the distal tip of the catheter 260 and contacted the saline 252. As a result, more current flows through the wire 240, saline 252, and the bowl 250 due to the lower resistance of the wire 240. This increased current is detected by the multimeter 220. These simulations and test results demonstrate that a conductive temporary connection or detachment zone, placed initially in an insulative environment or chamber and exiting the distal tip of the catheter to be part of a conductive path, triggers a change in an electrical parameter, such as current, through the circuit. This change can be used to activate an indicator to notify a user or serve as a signal for a control circuit.

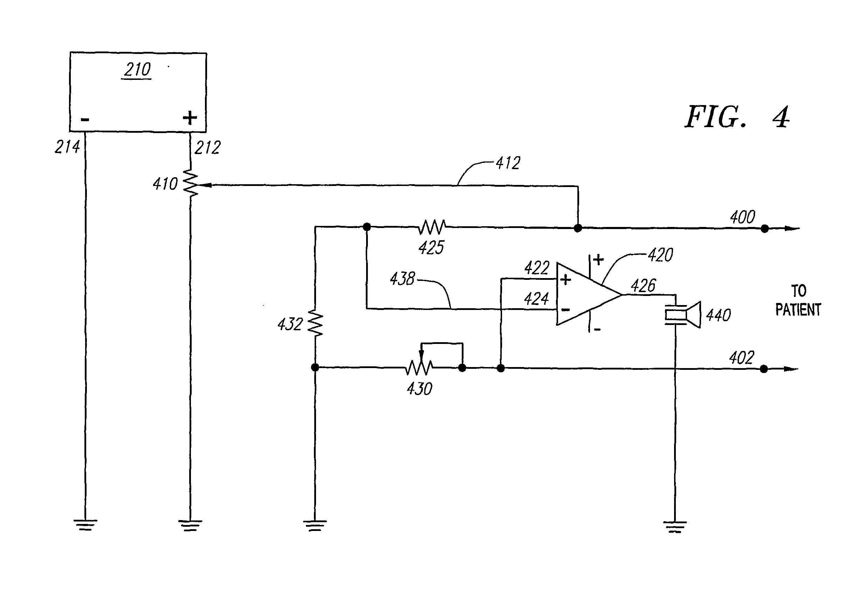

For example, **FIG. 4** illustrates one embodiment of a monitoring system 160 that utilizes a comparison circuit 420 and a buzzer 450 to indicate the position of the temporary connection. In this embodiment, the positive output 212 of the AC generator 210 is coupled to a patient lead 400 through a resistor 410 (e.g., a 5 KΩ variable resistor) and a wire 412. The positive output 212 is also coupled to a negative or reference input 424 of a comparator 420, such as an operational amplifier, through a resistor 425 and a wire 428. Thus, both the AC generator 210 and the comparator 420 are connected to the first patient lead 400. A positive input 422 of the comparator 420 is coupled to a second patient lead 402 and to the negative input 426 of the comparator through resistors 430 and 432, forming a feedback loop. As a result, when the patient leads 400 and 402 are connected to a patient body to complete the circuit (e.g., the body is one of four legs of a Wheatstone bridge) and the current is provided to the comparator input 422 via the feedback loop.

The reference value or threshold of the comparator 420 can be set so that the initial trickle current or initial state corresponding to the temporary connection 230 not contacting the body or blood in an aneurysm results in a low output 426. At this stage, the low output 426 of the comparator 420 would not activate an indicator, such as a buzzer 440. As the temporary connection 130 advances further and exits the catheter or contacts the body or blood, then the input current 422 is larger than the reference or threshold 424. As a result, the output 426 will change from low to high, and the output 426 can activate the buzzer 440 to inform a user that the implant 140 is properly positioned and can be detached from a delivery system. The user may then manually initiate detachment of the implant or detachment can be automatically initiated with a controller.

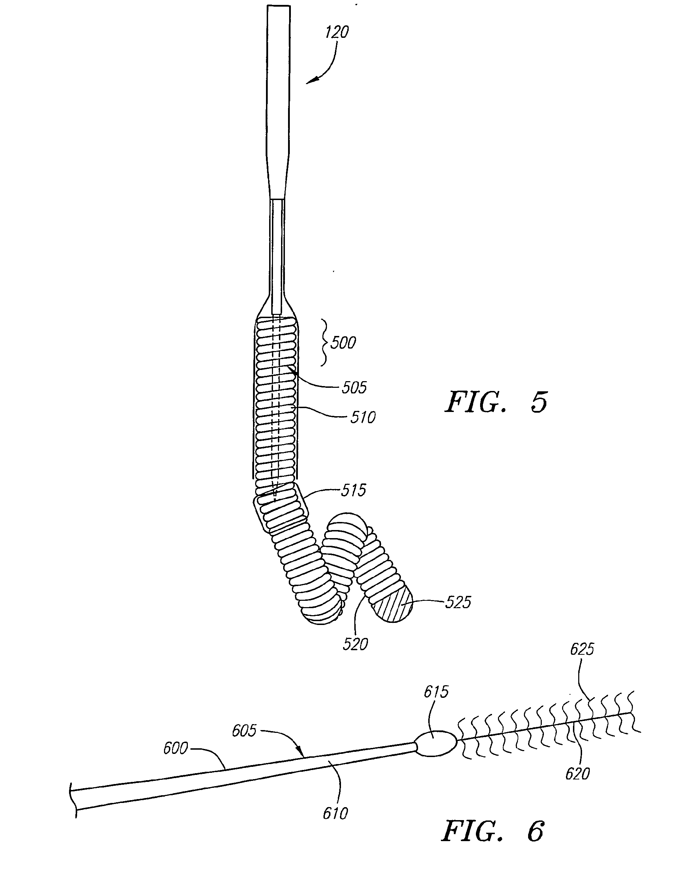

Referring to **FIG. 5**, the wire 120 disposed in the catheter 110 may be a stainless steel wire laminated with Teflon^{®}. An exemplary wire 120 has a diameter of approximately 0.010-0.020 inch (0.254-0.508 mm) and a length of about 50-300 cm. A first bonding location 500 may be covered with an insulating Teflon laminate 505, which encapsulates the underlying portion of wire 120 to prevent contact with the blood when being inserted through the catheter 110. A stainless steel coil 510 is attached or bonded to the wire 120 at the first bonding location 500. For example, the stainless steel coil 510 can be soldered, welded or adhered to the wire 120. The distal end of stainless steel coil 500 is attached to the distal end of the wire 120 and to the proximal end of an.implant 140, such as a platinum GDC coil, at a second bonding location 515.

One exemplary GDC coil forms a spiral or helix typically between 2 to 10 mm. in diameter. The helical envelope formed by a secondary coil 520 may be cylindrical or conical. Like the wire 120 and the stainless steel coil 510, the coil 520 is between approximately 0.010 and 0.020 inch (0.254-0.508 mm) in diameter. The coil 520 is soft and its overall shape can be deformed. When inserted within the catheter 110, the coil 520 is straightened to lie axially within the catheter 110. Once disposed out of the distal tip 116 of the catheter 110, the coil 520 forms a deformable shape and may be shaped to the interior shape of the aneurysm.

Referring to **FIG. 6**, a further exemplary implant 140 is a wire 600 that has an end portion 605 covered with a Teflon^{®} laminate 610. The wire 600 is attached by means of a mechanical coupling 615 to a platinum coil 620. The platinum coil 620 has a plurality of filaments 625 extending there from. For example, in a small vessel, hair 625 lengths of up to 1 mm can be utilized. The hairs 625 pack, fill or at least impede blood flow or access in the vascular cavity. The coil 620 has sufficient length and flexibility so that it can be inserted or coiled loosely into an aneurysm or other vascular cavity.

The tip 104 may also be mechanically separated from the wire 120 by various other temporary connections 130. One alternative connection 130 is a spring loaded mechanical clasp (not shown). The clasps are retained on the tip as long as the clasps remain inside of the catheter 110, but spring open and release tip 104 when extended from the catheter. A further alternative connection 130 is a nonresilient mechanical ball and clasp capturing mechanism. In yet a further embodiment, the wire 120 and the tip portion 625 screw into each other and can be unscrewed from each other by rotation of the catheter or wire with respect to tip 104. Persons of ordinary skill in the art will recognize that other mechanical detachment configurations can be utilized.

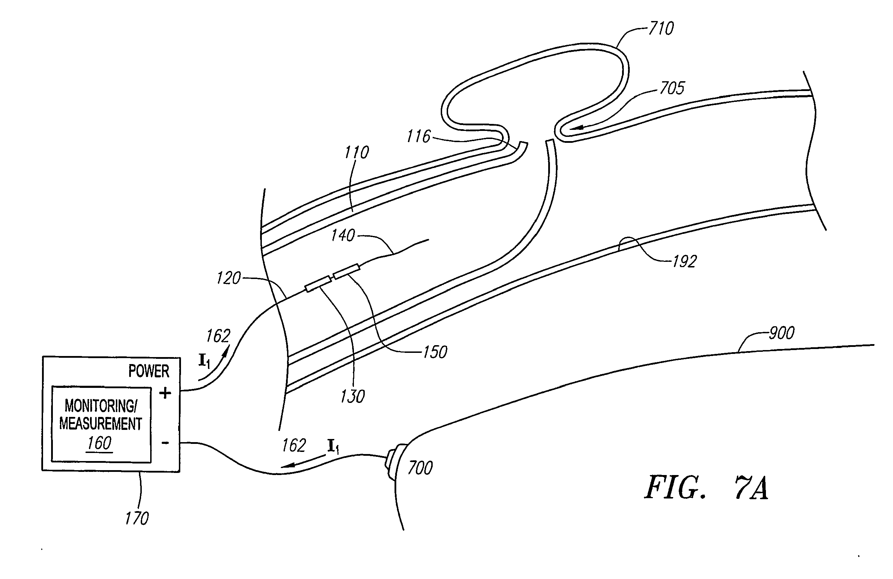

In use, as shown in **FIGS. 7A-C**, the coil implant 140 is used as an electrical anode while the cathode is a skin electrode 700 typically conductively applied to the groin or scalp. In an alternative embodiment, the catheter 110 is supplied with an end electrode coupled to an electrical conductor disposed along the length of catheter 110. A wire is led back to voltage source 170 so that the ring electrode is used as the cathode instead of an exterior skin electrode 700. This specification, however, refers to a portion of the body serving as a cathode for purposes of explanation and illustration.

**FIGS. 7A-C** illustrate the wire 120, temporary connection or detachment zone 130, insulative element 150 and coil 140 components being advanced through the catheter 110. The distal end 116 of catheter 110 is placed into a neck 705 of the aneurysm 710. In **FIG. 7A****,** the components are still contained within the insulative catheter 110. Thus, the electrical condition or current 162 is the smaller, trickle current I₁. When the coil implant 140 is disposed within the catheter 110, it lies along the longitudinal lumen 112 defined by catheter 110.

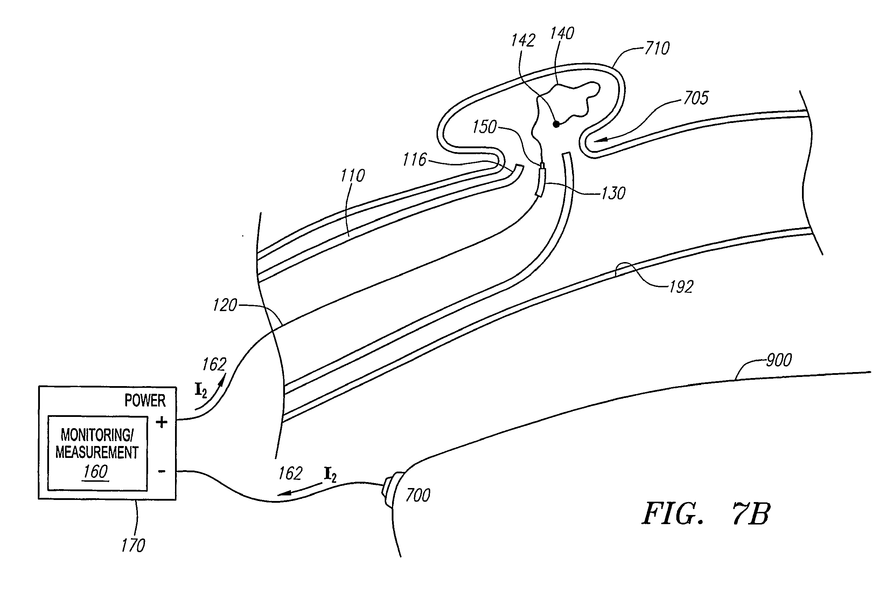

**FIG. 7B** shows the wire 120 being advanced, thereby feeding the tip 142 of the coil 140 into the aneurysm 710, and the bonding location or temporary connection reaching the distal tip 116 of the catheter 110. As a result, a portion of the stainless steel coil 510 (**FIG. 5**) of the temporary connection 130 is exposed beyond the distal tip 116 of catheter 110. The temporary connection 130 contacts blood in the aneurysm 710, thereby completing a circuit with less resistance. Thus, the current increases from I₁ 162 to I₂ 164, and this change in electrical condition indicates that the temporary connection or detachment zone 130 has reached or passed the distal tip 116 of the catheter. Thus, the coil 140 is properly positioned and can be detached.

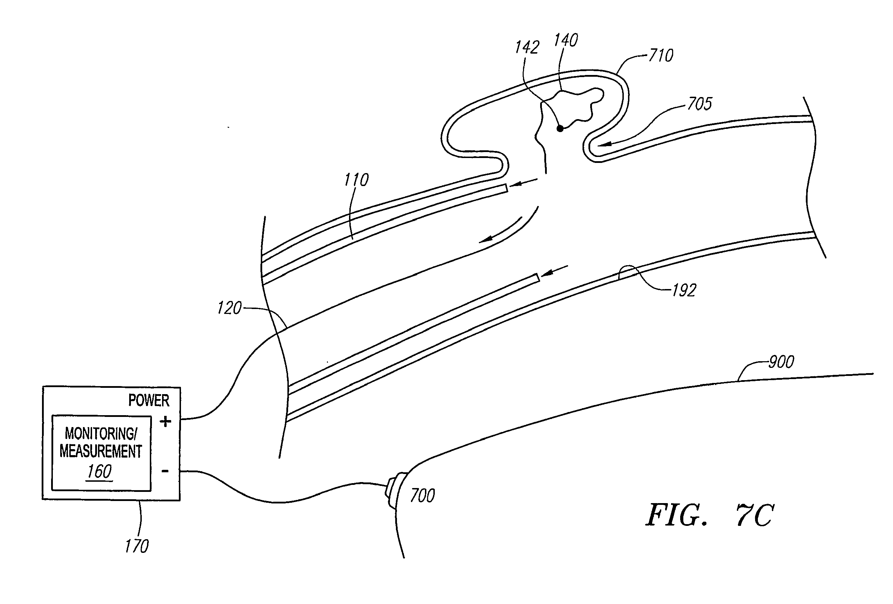

In response to this change in the monitored electrical condition, the monitoring or measuring system 160 provides an output signal to a user. The user can manually initiate detachment of the device, or the output signal can automatically trigger the power supply 170 to provide a direct current (DC) through the wire 120 to the temporary connection 130. An occlusion is eventually formed as a result of the reduced blood flow to the aneurysm. As shown in **FIG. 7C****,** after the aneurysm is occluded, the tip 142 and coil implant 140 are detached from the wire 120 by electrolytic disintegration of at least one portion of stainless steel coil 510 of the detachment zone or bond 130. For example, the coil 140 can be detached from the temporary connection 130 by continued application of current for a predetermined time when the stainless steel 510 is exposed to blood; or by movement of the wire 120 to expose stainless steel 510 to blood followed by continued current application for a predetermined time. In the illustrated embodiment this is accomplished by continued application of current until the total time of current application is almost approximately four minutes.

As a result, at least one portion of stainless steel coil 510 will be dissolved through by electrolytic action, typically within 2 minutes, usually less than one minute.

After separation by electrolytic disintegration, the wire 120, catheter 110 and the remaining portion of stainless steel coil 510 still attached to the wire 120 are removed from vascular space 192, leaving the coil 140 in the occluded aneurysm 710. It will be appreciated that the time of disintegration may be varied by altering the dimensions of the portions of the wire and/or the current.

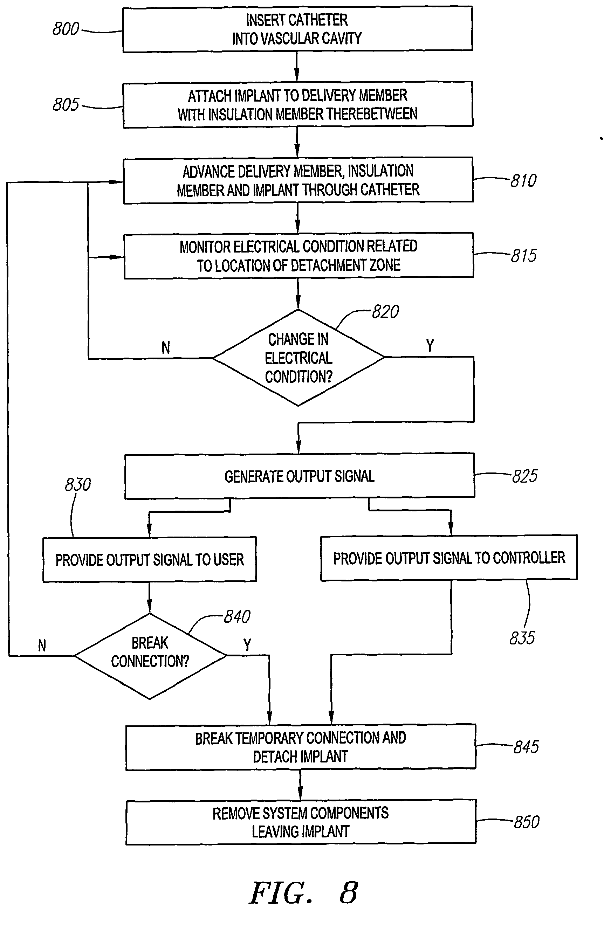

As previously discussed, different temporary connections may utilize different mechanisms to initiate breaking of the temporary connection. Further, various other controllable coils and implants can be used with the invention. Referring to **FIG. 8**, a method of monitoring a position of an implant is shown. Various steps have been previously described with respect to the operation and function of the system related to **FIGS. 1-8**.

In stage 800, a catheter is inserted into a vascular cavity. In stage 805, an implant, such as a vaso-occlusive device, a GDC, a stent or another suitable implant, is attached to a delivery member having a temporary connection. An insulative member may be placed between the temporary connection and the implant. In stage 810, the delivery member with the temporary connection, the insulative member and the implant are advanced through the lumen of the catheter. In stage 815, an electrical condition related to the location of the temporary connection is monitored with a sensor or a suitable measurement device.

In stage 820, a determination of whether the electrical condition has changed is made. If the electrical condition has changed, then the temporary connection has reached a predetermined location, e.g., the distal end or tip of the catheter, and the method proceeds to stage 825. If the electrical condition has not changed, then the components are advanced further into the catheter in stage 810 and the system continues to monitor the electrical condition at stage 815.

Continuing with stage 825, an output signal indicating a change in electrical condition is generated. The output signal indicates that the temporary connection and the implant are properly positioned. The output signal can be provided to a user in stage 830 or to a controller at stage 835. If the output signal is provided to a user at stage 830, then the user can decide whether to break the temporary connection and detach the implant at stage 840. The user can also advance or adjust the delivery member as needed before breaking the connection. If the user decides to detach the implant, then in stage 845, the user initiates detachment of the implant by breaking the temporary connection.

If the output signal is provided to a controller in step 835, then the controller can be configured to initiate breaking of the temporary connection in step 845 immediately or after a delay, if necessary.

At stage 850, the system components can be removed, leaving the implant to occlude the aneurysm site.

Having described a system and a method for monitoring the position of a implant both inside and outside a delivery catheter, persons of ordinary skill in the art will recognize that the above system can be modified in various ways to perform the same monitoring functions. For example, the invention can be used with various implants, and a vaso-occlusive GDC coil is merely illustrative of various suitable implants. Further, other monitoring systems and configurations can be utilized to determine an electrical condition, such as current, voltage, resistance, impedance, and other conditions as needed, to monitor the position of a temporary connection or detachment zone and an implant.

## Claims

1. A system for positioning an implant (140) in a body, the system including:
- a catheter (110) having a proximal end (114) and a distal end (116) and being insertable within a vascular cavity within the body,
- a delivery member (120),
- a temporary connection (130) joining the implant (140) and a distal end (124) of the delivery member (120), the temporary connection (130), the delivery member (120), and the implant (140) being configured for advancement through the catheter (110),and
- an electrical measurement device (160), the electrical measurement device (160) being configured to monitor an electrical condition (162) related to a position of the temporary connection (130), while the temporary connection (130) is joined to the delivery member (120) and to the implant (140), the electrical condition (162) changing when the temporary connection (130) reaches a predetermined location (180) as the delivery member (120) is advanced, through the catheter (110), the electrical measurement device (160) being further configured to generate an output signal in response to the changed electrical condition (162), the output signal indicating that the temporary connection (130) has reached the predetermined location (180)
**characterized in that**
the system further comprises an insulative member (150) positioned between and connecting the implant (140) and the temporary connection (130), the insulative member (150) being also configured for advancement through the catheter (110), together with the delivery member (120), the temporary connection (130), the delivery member (120), and the implant (140).

2. The system of claim 1, the delivery member (120) comprising a delivery wire.

3. The system of claim 1, the delivery member (120) comprising a tubular body.

4. The system of claim 1, the temporary connection (130) comprising an electrolytic connection.

5. The system of claim 4, further comprising a power supply (170), the electrolytic connection being breakable by current provided by the power supply (170) through the delivery member (120) and the temporary connection (130) corroding a portion of the temporary connection (130).

6. The system of claim 5, the portion of the temporary connection (130) that can be corroded comprising a stainless steel portion of the delivery member (120) for exposure to blood in the vascular cavity during use.

7. The system of claim 5, the electrical measurement, device (160) being included in the power supply (170).

8. The system of claim 5, the electrical measurement device (160) being separate from the power supply (170).

9. The system of claim 1, the temporary connection (130) comprising a breakable temporary mechanical connection.

10. The system of claim 1, the temporary connection (130) comprising a temporary connection that is breakable by application of heat.

11. The system of claim 1, the temporary connection (130) comprising a temporary connection that is breakable by application of Radio Frequency (RF) radiation.

12. The system of claim 1, the temporary connection (130) comprising a temporary connection that is hydraulically breakable.

13. The system of claim 1, the electrical measurement device (160) being configured to generate an output signal comprising a visual signal.

14. The system of claim 1, the electrical measurement device (160) being configured to generate an output signal comprising an audio signal.

15. The system of claim 1, the electrical measurement device (160) being configured to provide the output signal to a user, the temporary connection (130) being breakable response to user input.

16. The system of claim 1, the electrical measurement device (160) being configured to provide the output signal to a controller, the temporary connection (130) being breakable response to the controller.

17. The system of claim 1, the predetermined location (180) comprising the distal end (116) of the catheter (110).

18. The system of claim 17, the catheter (110), the temporary connection (130) and the implant (140) being configured such that the electrical condition (162) changes when the temporary connection(130) reaches the distal end (116) of the catheter(110).

19. The system of claim 19, the catheter (111), the temporary connection (130) and the implant (140) being configured such that the electrical condition (162) changes when the temporary connection (130) exits the distal end (116) of the catheter (110).

20. The system of claim 1, the electrical measurement device (160) being configured to compare a reference current with a second current that is generated when the temporary connection (130) reaches the predetermined location (180).

21. The system of claim 1, the electrical measurement device(160) including a comparison circuit configured to compare a threshold current to a current measured by the electrical measurement device (160), the comparison circuit being configured to generate an output indicating whether the temporary connection (130) has reached the predetermined location (180).

22. The system of claim 1, wherein the temporary connection is non-conductive, further comprising a conductive wire connected between the electrical measurement device (160) and the distal end (116) of the catheter (110), the electrical measuring device (160) detecting an electrical condition (162) related to a position of the temporary connection (130) in the catheter (110) through the conductive wire.

## Patentansprüche

1. System zur Positionierung eines Implantats (140) in einem Körper, wobei das System umfasst:
- einen Katheter (110) mit einem proximalen Ende (114) und einem distalen Ende (116), der innerhalb eines vaskulären Hohlraums innerhalb des Körpers eingefügt werden kann,
- ein Zufuhrbauteil (120),
- eine zeitweise Verbindung (130), die das Implantat (140) und ein distales Ende (124) des Zufuhrbauteils (120) verbindet, wobei die zeitweise Verbindung (130), das Zufuhrbauteil (120) und das Implantat (140) zum Voranschieben durch den Katheter (110) gestaltet sind, und
- eine elektrische Messvorrichtung (160), wobei die elektrische Messvorrichtung (160) gestaltet ist zur Überwachung eines elektrischen Zustands (162), der eine Position der zeitweisen Verbindung (130) betrifft, während die zeitweise Verbindung (130) das Zufuhrbauteil (120) und das Implantat (140) verbindet, wobei sich der elektrische Zustand (162) verändert, wenn die zeitweise Verbindung (130) einen vorher bestimmten Ort (180) erreicht, wenn sich das Zufuhrbauteil (120) durch den Katheter (110) vorwärts bewegt wird, und die elektrische Messvorrichtung (160) weiterhin gestaltet ist zur Erzeugung eines Ausgabesignals als Antwort auf den veränderten elektrischen Zustand (162), wobei das Ausgabesignal anzeigt, dass die zeitweise Verbindung (130) den vorherbestimmten Ort (180) erreicht hat,
**dadurch gekennzeichnet, dass**
das System weiterhin ein isolierendes Bauteil (150) umfasst, welches sich zwischen dem Implantat (140) und der zeitweisen Verbindung (130) befindet und diese verbindet, wobei das isolierende Bauteil (150) auch zum vorwärts Verschieben durch den Katheter (110) gestaltet ist, zusammen mit dem Zufuhrbauteil (120), der zeitweisen Verbindung (130), dem Zufuhrbauteil (120) und dem Implantat (140).

2. System nach Anspruch 1, wobei das Zufuhrbauteil (120) einen Zufuhrdraht umfasst.

3. System nach Anspruch 1, wobei das Zufuhrbauteil (120) einen röhrenförmigen Körper umfasst.

4. System nach Anspruch 1, wobei die zeitweise Verbindung (130) eine elektrolytische Verbindung umfasst.

5. System nach Anspruch 4, weiterhin umfassend eine Energieversorgung (170), wobei die elektrolytische Verbindung durch Strom unterbrochen werden kann, wenn Strom durch die Energieversorgung (170) bereitgestellt wird durch das Zufuhrbauteil (120) und wobei die zeitweise Verbindung (130) einen Abschnitt der zeitweisen Verbindung (130) korrodiert.

6. System nach Anspruch 5, wobei der Abschnitt der zeitweisen Verbindung (130), der korrodieren kann, einen rostfreien Stahlabschnitt des Zufuhrbauteils (120) zur Einwirkung des Bluts in dem vaskulären Hohlraum während der Verwendung umfasst.

7. System nach Anspruch 5, wobei die elektrische Messvorrichtung (160) in die Energieversorgung (170) eingeschlossen ist.

8. System nach Anspruch 5, wobei die elektrische Messvorrichtung (160) von der Energieversorgung (170) getrennt ist.

9. System nach Anspruch 1, wobei die zeitweise Verbindung (130) eine zeitweise mechanische Verbindung umfasst, die unterbrochen werden kann.

10. System nach Anspruch 1, wobei die zeitweise Verbindung (130) eine zeitweise Verbindung umfasst, die durch Anwendung von Hitze unterbrochen werden kann.

11. System nach Anspruch 1, wobei die zeitweise Verbindung (130) eine zeitweise Verbindung umfasst, die durch die Anwendung von hochfrequenter (RF) Strahlung unterbrochen werden kann.

12. System nach Anspruch 1, wobei die zeitweise Verbindung (130) eine zeitweise Verbindung umfasst, die hydraulisch unterbrochen werden kann.

13. System nach Anspruch 1, wobei die elektrische Messvorrichtung (160) zur Erzeugung eines Ausgabesignals gestaltet ist, welches ein visuelles Signal umfasst.

14. System nach Anspruch 1, wobei die elektrische Messvorrichtung (160) zur Erzeugung eines Ausgabesignals gestaltet ist, welches ein Audiosignal umfasst.

15. System nach Anspruch 1, wobei die elektrische Messvorrichtung (160) zur Bereitstellung eines Ausgabesignals an einen Benutzer gestaltet ist, wobei die zeitweise Verbindung (130) auf eine Eingabe des Benutzers hin unterbrochen werden kann.

16. System nach Anspruch 1, wobei die elektrische Messvorrichtung (160) zur Bereitstellung des Ausgabesignals an eine Steuerung gestaltet ist, wobei die zeitweise Verbindung (130) als Antwort auf die Steuerung unterbrochen werden kann.

17. System nach Anspruch 1, wobei der vorherbestimmte Ort (180) das distale Ende (116) des Katheters (110) umfasst.

18. System nach Anspruch 17, wobei der Katheter (110), die zeitweise Verbindung (130) und das Implantat (140) so gestaltet sind, dass sich der elektrische Zustand (162) ändert, wenn die zeitweise Verbindung (130) das distale Ende (116) des Katheters (110) erreicht.

19. System nach Anspruch 17, wobei der Katheter (111), die zeitweise Verbindung (130) und das Implantat (140) so gestaltet sind, dass sich der elektrische Zustand (162) ändert, wenn die zeitweise Verbindung (130) an dem distalen Ende (116) des Katheters (110) austritt.

20. System nach Anspruch 1, wobei die elektrische Messvorrichtung (160) zum Vergleich eines Referenzstroms mit einem zweiten Strom gestaltet ist, welcher erzeugt wird, wenn die zeitweise Verbindung (130) den vorbestimmten Ort (180) erreicht.

21. System nach Anspruch 1, wobei die elektrische Messvorrichtung (160) eine Vergleichsschaltung einschließt, die gestaltet ist zum Vergleich eines Schwellwertstroms mit einem Strom, der durch die elektrische Messvorrichtung (160) gemessen wird, wobei die Vergleichsschaltung gestaltet ist zur Erzeugung einer Ausgabe, die anzeigt, ob die zeitweise Verbindung (130) den vorbestimmten Ort (180) erreicht hat.

22. System nach Anspruch 1, wobei die zeitweise Verbindung nicht leitend ist und weiterhin einen leitenden Draht umfasst, der zwischen der elektrischen Messvorrichtung (160) und dem distalen Ende (116) des Katheters (110) verbunden ist, wobei die elektrische Messvorrichtung (160) einen elektrischen Zustand (162) wahrnimmt, der sich auf eine Position der zeitweisen Verbindung (130) in dem Katheter (110) durch den leitfähigen Draht bezieht.

## Revendications

1. Un système pour positionner un implant (140) dans un corps, le système comprenant :
- un cathéter (110) ayant une extrémité proximale (114) et une extrémité distale (116) et qui peut être inséré au sein d'une cavité vasculaire à l'intérieur du corps,
- un organe de délivrance (120),
- une connexion temporaire (130) reliant l'implant (140) et une extrémité distale (124) de l'organe de délivrance (120), la connexion temporaire (130), l'organe de délivrance (120) et l'implant (140) étant configurés de manière à pouvoir avancer au travers du cathéter (110), et
- un dispositif de mesure électrique (160), le dispositif de mesure électrique (160) étant configuré pour surveiller une condition électrique (162) en relation avec une position de la connexion temporaire (130), lorsque la connexion temporaire (130) est reliée à l'organe de délivrance (120) et à l'implant (140), la condition électrique (162) changeant lorsque la connexion temporaire (130) atteint un emplacement prédéterminé (180) au cours de l'avancement de l'organe de délivrance (120) au travers du cathéter (110), le dispositif de mesure électrique (160) étant en outre configuré pour produire un signal de sortie en réponse à la condition électrique modifiée (162), le signal de sortie indiquant que la connexion temporaire (130) a atteint l'emplacement prédéterminé (180),
**caractérisé en ce que**
le système comprend en outre un organe isolant (150) positionné entre l'implant et la connexion temporaire (130) et connectant ceux-ci, l'organe isolant (150) étant également configuré pour pouvoir avancer au travers du cathéter (110), en même temps que l'organe de délivrance (120), la connexion temporaire (130), l'organe de délivrance (120) et l'implant (140).

2. Le système de la revendication 1, où l'organe de délivrance (120) comprend un fil de délivrance.

3. Le système de la revendication 1, où l'organe de délivrance (120) comprend un corps tubulaire.

4. Le système de la revendication 1, où la connexion temporaire (130) comprend une connexion électrolytique.

5. Le système de la revendication 4, comprenant en outre une alimentation en énergie (170), la connexion électrique pouvant être brisée par un courant délivré par l'alimentation en énergie (170) au travers de l'organe de délivrance (120) et la connexion temporaire (130) corrodant une partie de la connexion temporaire (130).

6. Le système de la revendication 5, où la partie de la connexion temporaire (130) qui peut être corrodée comprend une partie en acier inoxydable de l'organe de délivrance (120) destinée à être exposée au sang dans la cavité vasculaire pendant l'utilisation.

7. Le système de la revendication 5, où le dispositif de mesure électrique (160) est inclus dans l'alimentation en énergie (170).

8. Le système de la revendication 5, où le dispositif de mesure électrique (160) est séparé de l'alimentation en énergie (170).

9. Le système de la revendication 1, où la connexion temporaire (130) comprend une connexion mécanique temporaire brisable.

10. Le système de la revendication 1, où la connexion temporaire (130) comprend une connexion temporaire qui peut être brisée par application de chaleur.

11. Le système de la revendication 1, où la connexion temporaire (130) comprend une connexion temporaire qui peut être brisée par application d'un rayonnement radiofréquence (RF).

12. Le système de la revendication 1, où la connexion temporaire (130) comprend une connexion temporaire qui peut être brisée hydrauliquement.

13. Le système de la revendication 1, où le dispositif de mesure électrique (160) est configuré pour produire un signal de sortie comprenant un signal visuel.

14. Le système de la revendication 1, où le dispositif de mesure électrique (160) est configuré pour produire un signal de sortie comprenant un signal audio.

15. Le système de la revendication 1, où le dispositif de mesure électrique (160) est configuré pour délivrer le signal de sortie à un utilisateur, la connexion temporaire (130) pouvant être brisée en réponse à une entrée de l'utilisateur.

16. Le système de la revendication 1, où le dispositif de mesure électrique (160) est configuré pour délivrer le signal de sortie à un contrôleur, la connexion temporaire (130) pouvant être brisée en réponse au contrôleur.

17. Le système de la revendication 1, où l'emplacement prédéterminé (180) comprend l'extrémité distale (116) du cathéter (110).

18. Le système de la revendication 17, où le cathéter (110), la connexion temporaire (130) et l'implant (140) sont configurés de sorte que la condition électrique (162) change lorsque la connexion temporaire (130) atteint l'extrémité distale (116) du cathéter (110).

19. Le système de la revendication 17, où le cathéter (110), la connexion temporaire (130) et l'implant (140) sont configurés de sorte que la condition électrique (162) change lorsque la connexion temporaire (130) quitte l'extrémité distale (116) du cathéter (110).

20. Le système de la revendication 1, où le dispositif de mesure électrique (160) est configuré pour comparer un courant de référence avec un second courant qui est produit lorsque la connexion temporaire (130) atteint l'emplacement prédéterminé (180).

21. Le système de la revendication 1, où le dispositif de mesure électrique (160) inclut un circuit de comparaison configuré pour comparer un courant de seuil à un courant mesuré par le dispositif de mesure électrique (160), le circuit de comparaison étant configuré pour produire une sortie indiquant si la connexion temporaire (130) a ou non atteint l'emplacement prédéterminé (180).

22. Le système de la revendication 1, dans lequel la connexion temporaire est non conductrice, comprenant en outre un fil conducteur connecté entre le dispositif de mesure électrique (160) et l'extrémité distale (116) du cathéter (110), le dispositif de mesure électrique (160) détectant une condition électrique (162) en relation avec une position de la connexion temporaire (130) dans le cathéter (110) au travers du fil conducteur.
